(19)

Europäisches
Patentamt

European
Patent Office

Office européen
des brevets

(11) **EP 2 772 246 A1**

(12) **EUROPEAN PATENT APPLICATION**

(43) Date of publication:
**03.09.2014 Bulletin 2014/36**

(51) Int Cl.:
***A61K 8/36*** *(2006.01)*     ***A61Q 5/06*** *(2006.01)*

(21) Application number: **13157363.6**

(22) Date of filing: **01.03.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **The Procter & Gamble Company Cincinnati, OH 45202 (US)**

(72) Inventors:
 • **Flohr, Andreas**
  **61476 Kronberg im Taunus (DE)**

 • **Kripp, Thomas**
  **64407 Fränkisch-Crumbach (DE)**

(74) Representative: **Holmes, Rosalind**
  **Procter & Gamble Service GmbH**
  **Legal Innovation**
  **Patent Department**
  **Frankfurter Straße 145**
  **61476 Kronberg im Taunus (DE)**

(54) **Acidic composition for modifying the internal region of a hair shaft**

(57)     A cosmetic composition for modifying the internal region of a hair shaft where the composition comprises: (a) from about 1.1% to about 4.9% ethylenic monomer having a molecular weight of from about 50 g/mol to about 105 g/mole and is an acid; (b) a cosmetically acceptable carrier; and also where the composition has a pH of from about 2.6 to about 4.5.

EP 2 772 246 A1

**Description**

FIELD OF THE INVENTION

Cosmetic composition for modifying the internal region of a hair shaft.

BACKGROUND OF THE INVENTION

Methods for chemically modifying the internal region of the hair shaft are known. WO2009/088520A, WO2012/100006A, WO2012/100007A and EP2295029A describe the use of ethylenic monomers to chemically modify the internal region of the hair shaft - in particular the ethylenic molecules may bond to the hair and/or to each other to form larger molecules e.g. polymers inside the hair. This increases the rigidity of the hair via the modification of the internal structure of the hair shaft, which provides styling advantages e.g. allowing style formation or increased volume and style retention for longer periods of time. 3-sulfopropyl(meth)acrylate potassium salt (3-SPA) is a known monomer for use in chemically modifying the internal region of the hair shaft and has a M.Wt. of 232.3 g/mol. US3,472,243 discloses a process for strengthening and conditioning over-porous hair by polymerizing in said hair a water soluble polymerizable monomeric vinylic compound having acid groups and then bridging the polymer chains so formed with a bridging agent to reduce the water solubility of the polymer chains. US3,472,243 states that a pH of between 8 and 9 or 10 is particularly suitable.

There is a need for improvements in the modification of the internal region of the hair shaft. Whilst certain previous methods may provide satisfactory results to the hair shaft, there is a constant need for providing methods resulting in further improved performance, efficacy, and/or efficiency. There is a need for improving the durability of the treatment such that the benefits last for a longer time and for tailoring the reactivity of the active(s). There is also a need for providing more environmentally friendly and sustainable methods as well as making the method more economically viable and available to a greater breadth of consumer type. In addition there is a need for better ensuring that the results are more predictable and reducing the variability of the end result between consumers with different hair types.

SUMMARY OF THE INVENTION

According to a first aspect, the present invention relates to a cosmetic composition for modifying the internal region of a hair shaft, wherein the composition comprises:

a) from about 1.1% to about 4.9% ethylenic monomer, wherein the ethylenic monomer has a molecular weight of from about 50 g/mol to about 105 g/mole, and wherein the ethylenic monomer is an acid;
b) a cosmetically acceptable carrier;
wherein the composition has a pH of from about 2.6 to about 4.5.

According to a second aspect, the present invention relates to a method for modifying the internal region of a hair shaft, wherein the method comprises: (A) mixing the composition according to the first aspect with an initiator formulation to form a mixture; and then (B) applying the mixture onto hair.

According to a third aspect, the present invention relates to a process for preparing a mixture for applying onto hair, wherein the process comprises mixing a composition, according to the first aspect, with an initiator formulation being in solid form.

According to a fourth aspect, the present invention relates to a kit comprising (i) the composition of the first aspect; (ii) an initiator formulation.

According to a fifth aspect, the present invention relates to the use of the composition according to the first aspect applying onto hair.

BRIEF DESCRIPTION OF THE DRAWINGS

Fig. 1: Shows the percentage conversion of ethylenic monomer into polymer (disappearance of monomer) over time as measured by $^1$H-NMR (proton nuclear magnetic resonance). Different pHs were compared as well as two different monomers.

Fig. 2: Shows the percentage conversion of ethylenic monomer into polymer (disappearance of monomer) over time as measured by $^1$H-NMR. Different pHs were compared for compositions comprising acrylic acid and the experiments were done in a nitrogen gas atmosphere.

Fig. 3: Shows the percentage conversion of ethylenic monomer into polymer (disappearance of monomer) over time as measured by $^1$H-NMR. For compositions comprising acrylic acid, the effect of the presence of a buffer system was tested.

Fig.4: Shows the M.Wt of polymer formed for different compositions measured by gel permeation chromatography (GPC). Compositions respectively comprising the ethylenic monomers 3-SPA and acrylic acid are compared.

Fig.5: Shows the M.Wt of polymer formed for different compositions measured by GPC. Compositions comprising the ethylenic monomer acrylic acid are employed and the effect of different pHs is compared.

Fig.6: Shows the M.Wt of polymer formed for different compositions measured by GPC. The effect of a buffer system is tested.

Fig.7: Shows the results of an Add On experiment comparing the mean weight difference of hair swatches treated with a composition pursuant to the invention and a composition not pursuant to the invention.

## DETAILED DESCRIPTION OF THE INVENTION

### General and definitions

In all embodiments of the present invention, all percentages are by weight of the total composition (or formulation), unless specifically stated otherwise. All ratios are weight ratios, unless specifically stated otherwise. All ranges are inclusive and combinable. The number of significant digits conveys neither a limitation on the indicated amounts nor on the accuracy of the measurements. All numerical amounts are understood to be modified by the word "about" unless otherwise specifically indicated. Unless otherwise indicated, all measurements are understood to be made at 25°C and at ambient conditions, where "ambient conditions" means conditions under about 1 atmosphere of pressure and at about 50% relative humidity. All weights as they pertain to listed ingredients are based on the active level and do not include carriers or by-products that may be included in commercially available materials, unless otherwise specified. Herein "min" means "minute" or "minutes". Herein "mol" means mole. Herein "g" following a number means "gram" or "grams". Herein, "comprising" means that other steps and other ingredients which do not affect the end result can be added. This term encompasses the terms "consisting of" and "consisting essentially of". The compositions, methods, uses, kits, and processes of the present invention can comprise, consist of, and consist essentially of the elements and limitations of the invention described herein, as well as any of the additional or optional ingredients, components, steps, or limitations described herein.

The term "substantially free from" or "substantially free of" as used herein means less than about 1%, less than about 0.8%, less than about 0.5%, less than about 0.3%, or about 0%, by total weight of the composition or formulation.

"Hair," as used herein, means mammalian hair including scalp hair, facial hair and body hair, more preferably hair on the human head and scalp. "Hair shaft" means an individual hair strand and may be used interchangeably with the term "hair." "Internal region of the hair shaft," as used herein, means any non-surface portion of the hair shaft, including the inner portion of the cuticle, underneath the cuticle and the cortex. "Non-surface portion" may be understood to mean that portion of the hair that is not in direct contact with the outside environment.

"Cosmetically acceptable," as used herein, means that the compositions, formulations or components described are suitable for use in contact with human keratinous tissue without undue toxicity, incompatibility, instability, allergic response, and the like. All compositions and formulations described herein which have the purpose of being directly applied to keratinous tissue are limited to those being cosmetically acceptable.

"Derivatives," as used herein, includes but is not limited to, amide, ether, ester, amino, carboxyl, acetyl, acid, salt and/or alcohol derivatives of a given compound.

"Monomer," as used herein, means a discrete, non-polymerised chemical moiety capable of undergoing polymerisation in the presence of an initiator. "Ethylenic monomer," as used herein, means a chemical species that contains an olefinic carbon-carbon double bond (C=C) and is capable of undergoing polymerization in the presence of an initiator.

"Polymer," as used herein, means a chemical formed from the polymerisation of two or more monomers. The term "polymer" as used herein shall include all materials made by the polymerisation of monomers as well as natural polymers. Polymers made from only one type of monomer are called homopolymers. A polymer comprises at least two monomers. Polymers made from two or more different types of monomers are called copolymers. The distribution of the different monomers can be calculated statistically or block-wise - both possibilities are suitable for the present invention. Except if stated otherwise, the term "polymer" used herein includes any type of polymer including homopolymers and copolymers.

The term "M.Wt." or "MW" and the like as used herein means the molecular weight and refers to the number average M.Wt. unless otherwise stated.

"Chemically modify," or grammatical equivalents thereof, as used herein, means that a chemical moiety such as monomer and/or crosslinker and/or polymer, stably affixes to a second chemical moiety, for example, a keratin protein, another component of hair, and/or another monomer or crosslinker or polymer. Normally, "chemically modify" means stably affix via a covalent bond, unless otherwise stated.

"Separately packaged," as used herein, means any form of packaging that prevents one composition or formulation from coming into physical contact, or admixing, with a second composition or formulation.

"Kit," as used herein, means a packaging unit comprising a plurality of components i.e. a kit of parts. An example of a kit is, for example, a first composition and a separately packaged second composition. Another kit may comprise application instructions comprising a method and a composition/formulation.

As already discussed above, there is a constant need for improvements in the modification of the hair shaft. The invention described herein provides significant improvements versus available technologies. A particular advantage is the better penetration into the hair shaft results due to the lower M.Wt. of ethylenic monomer versus previous technologies. This means more monomer penetration into the hair and additional penetration into regions of the hair where higher M.Wt. monomer (due to steric hindrance, physical size etc) does not penetrate, therefore a higher "add-on" is achieved with the ethylenic monomer of the present invention. Also, a significantly higher M.Wt. polymer is formed with the compostion of the present invention compared to comparative compositions, thus better wash fastness and improved benefit durability results.

It is also possible to achieve tailored reactivity of the acidic monomer via partial neutralization. Since the lower M.Wt. monomer penetrates deeper into hair, the tailored reactivity also allows controlled penetration depths with changes in the degree of neutralization of the acidic monomer. Also, the tailored glass-transition temperature of the lower M.Wt., acidic monomer and thus rigidity of the resulting polymer inside hair can be tailored via changes in the degree of neutralization.

Another benefit of the present invention is that there is a reduced chance for monomer stability issues e.g. auto-polymerization as seen for ester-type of monomers that cleave under basic or acidic conditions. The present invention also provides significant economical and sustainability advantages because, for example, acrylic acid is a precursor of acrylate-based monomers therefore synthetic steps are skipped since the acrylic acid or similar low M.Wt. monomer does not need to be first converted to a different monomer prior to incorporation in the cosmetic composition. The incorporation of a buffering agent in the composition provides further benefits because the regulation of the pH of the composition within a specific range of acidity means that the acid form of the ethylenic monomer is constantly renewed.

### 1$^{st}$ Aspect: Composition

The first aspect relates to a cosmetic composition, or "composition" herein. The composition is for modifying the internal region of a hair shaft. In an embodiment, the modifying is forming a polymer inside the hair shaft. In an embodiment, the modifying is selected from the group consisting of: the formation of a polymer in the internal region of the hair shaft; the modification of the internal region of the hair shaft with a monomer and/or polymer; and combinations thereof. In an embodiment, the modifying is the forming of a polymer in the internal region of the hair shaft by *in situ* polymerisation, wherein the polymerisation that occurs is free radical polymerisation.

The composition comprises an ethylenic monomer. In an embodiment, the ethylenic monomer comprises a vinyl group. "Vinyl group" as used herein, means $H_2C=CH-R$. In an embodiment, the ethylenic monomer comprises an acrylate group or a methacrylate group. "Acrylate group" as used herein, means $H_2C=CH-C(O)O-R$. Acrylic acid, for example, comprises an acrylate group since R is hydrogen. "Methacrylate group" as used herein means $H_2C=C(CH_3)-C(O)O-R$. In an embodiment, the ethylenic monomer is selected from the group consisting of: methacrylic acid, tiglic acid, acrylic acid, and mixtures thereof. In an embodiment, the composition comprises at least one ethylenic monomer, selected from the group cited above, as the sole ethylenic monomer(s). In an embodiment, the sole ethylenic monomer is acrylic acid. The M.Wt. of the ethylenic monomer is important because of the need for the monomer to penetrate into the hair shaft prior to polymerisation. Large and/or bulky monomers would penetrate less easily into the hair shaft. The ethylenic monomer has a M.Wt. of from about 50 g/mole to about 105 g/mole. In an embodiment, the ethylenic monomer has a M.Wt. of from about 70 g/mole to about 100 g/mole, or from about 75 g/mol to about 95 g/mol, or from about 75 g/mole to about 90 g/mole. In an embodiment, the composition is substantially free of an ethylenic monomer that has a M.Wt. of below about 50 g/mole, or below about 60 g/mol, or above about 200 g/mol, or above about 500 g/mole. In an embodiment, the composition comprises from about 1.2%, or 1.3%, or 1.4%, or 1.6%, or 1.8%, or 2.0%, or 2.2%, or 2.4%, or 2.5% to about 4.8%, or 4.7%, or 4.6%, or 4.5%, or 4.3%, or 4.1%, or 4.0%, or 3.9%, or 3.8%, or 3.7%, or 3.6%, or 3.5% of ethylenic monomer. This amount may be the total amount of ethylenic monomer in the composition. In an embodiment, the sole ethylenic monomer is acrylic acid and the composition contains from about 3.0% to about 4.2% ethylenic monomer i.e. from about 3.0% to about 4.2% acrylic acid. In an embodiment, two or more different ethylenic monomers are present in the composition pursuant to the first aspect. The resultant polymers may be copolymers. In an embodiment, the composition is substantially free of any nitrogen-containing ethylenic monomers. In an embodiment, the composition is substantially free of a nitrogen-containing ethylenic monomer. In an embodiment, the nitrogen-containing ethylenic monomer is selected from the group consisting of: amides, cyanates, nitriles, and ammonium-group containing compounds. The ethylenic monomer is an acid. In an embodiment, the ethylenic monomer is not a carboxylic acid.

In an embodiment, the composition comprises an inhibitor compound selected from the group consisting of: 2-*tert*-butyl-4-hydroxy-anisole, 3-*tert*-butyl-4-hydroxy-anisole, and mixtures thereof. The inhibitor compound may be present in the composition in an amount of from about 1 milligram to about 1000 milligram per kilogram of the ethylenic monomer. Such amount may be the total amount of inhibitor compound being those selected from the group consisting of: *2-tert*-butyl-4-hydroxy-anisole, 3-*tert*-butyl-4-hydroxy-anisole. In an embodiment, the inhibitor compound is present in the composition in an amount of from about 50 milligram to about 800 milligram, or from about 100 milligram to about 500 milligram, or from about 100 milligram to about 300 milligram, or from about 100 milligram to about 200 milligram, per kilogram of ethylenic monomer. In an embodiment, the inhibitor compound is the mixture of 2-*tert*-butyl-4-hydroxy-anisole and 3-*tert*-butyl-4-hydroxy-anisole. In an embodiment, the mixture of *2-tert*-butyl-4-hydroxy-anisole and 3-*tert*-butyl-4-hydroxy-anisole comprises greater than about 50%, or greater than about 60%, or greater than about 80% 3-*tert*-butyl-4-hydroxy-anisole. The inhibitor compound functions as a polymerisation inhibitor i.e. it stabilises the ethylenic monomer so that it does not polymerise prior to contact with an inhibitor. The inhibitor compound provides excellent performance in that the ethylenic monomer is effectively stabilised and also when the ethylenic monomer exposed to an initiator, the ethylenic monomer polymerises efficiently and quickly. In an embodiment, the composition is substantially free of a polymer derived from the polymerisation of ethylenic monomers. Other polymerisation inhibitors used for stabilising ethylenic monomers are known in the art e.g. 4-methoxy phenol. In an embodiment, the composition comprises less than about 500 ppm, or less than about 400 ppm, or less than about 300 ppm, or less than about 200 ppm, or less than about 100 ppm, or less than about 50 ppm, or less than about 10 ppm, of 4-methoxy phenol. In an embodiment, the composition is substantially free of a polymerisation inhibitor, with the exception of an inhibitor compound selected from the group consisting of: 2-*tert*-butyl-4-hydroxy-anisole, 3-*tert*-butyl-4-hydroxy-anisole, and mixtures thereof.

The composition may comprise a crosslinker having a M.Wt. suitable to penetrate the hair shaft. In an embodiment, the composition is substantially free of a crosslinker.

The composition may comprise a viscosity-increasing agent. Viscosity is important when the composition is in the form of gel, cream, lotion, emulsion and the like because it prevents the composition from sliding off the skin and/or hair. However, lower viscosities allow actives to penetrate/diffuse more easily e.g. into the internal region of the hair shaft. The viscosity of the composition when it is in the form of a gel may be from about 500 mPa·s to about 15000 mPa·s, or from about 1000 mPa·s to about 10000 mPa·s, or from about 1500 mPa·s to about 7500 mPa·s, or from about 2000 mPa·s to about 5000 mPa·s, measured with a Brookfield Viscosimeter RVDV III Ultra CP 52 at 25 °C and 1 rpm. The viscosity-increasing agent may be selected from the group consisting of non-ionic thickeners, cationic thickeners, anionic thickeners, amphoteric thickeners, and mixtures thereof. The viscosity-increasing agent may be present in the composition in an amount of from about 0.1 % to about 10%, or from about 0.2% to about 5.0%. In an embodiment, the composition comprises a non-ionic or anionic thickener (or mixtures thereof), which is in view of the typically anionic chemistry of any polymerised ethylenic monomer. Non-ionic or anionic thickeners are less likely to interact directly with any formed polymer and hence the formation of insoluble complexes or precipitates is also less likely. In an embodiment, the viscosity-increasing agent is stable at the required pH and does not substantially affect the active levels of ethylenic monomer. The viscosity-increasing agent may be a cross-linked or a non-crosslinked polymer. In an embodiment, the viscosity-increasing agent is a hydrophobically-modified polyacrylate polymer. The composition may comprise from about 0.5% to about 1.5% of the hydrophobically-modified polyacrylate polymer, by total weight of the composition. Suitable hydrophobically-modified polyacrylate polymers include: acrylates/C10-C30 alkylacrylates copolymers such as Ultrez® 20/21 from Lubrizol, and Permulen® TR1 from Lubrizol; acrylates/beheneth-25 methacrylate copolymers such as Aculyn® 28 from Rohm & Haas; acrylates/ceteth-20 itaconate copolymers such as Structure® 3001 or 2001 from Akzo Nobel. In an embodiment, the viscosity-increasing agent is a non-crosslinked associative thickening polymer. The composition or oxidising formulation may comprise from about 0.5% to about 3% of the non-crosslinked associative thickening polymer, by total weight of the composition. Suitable associative thickeners include polyurethane-based polymers such as polyurethane-30 e.g. LuvigelSTAR® from BASF. Also EO-PO-block copolymers may be useful, for example Pluronics® from BASF. In an embodiment, the viscosity-increasing agent comprises at least one polysaccharide. In an embodiment, the composition comprises a heteropolysaccharide. The total polysaccharide present in the composition may be from about 0.2% to about 5%, or from about 0.5% to about 4%, by total weight of the composition. Suitable polysaccharides and heterosaccharides include starches and derivatives thereof, e.g. mono- or diesters with phosphoric acid, cellulose types and their derivatives, xanthan gums, carrageenans. Preferred heteropolysaccharides include xanthan gum such as Keltrol® T from Kelco, and Natrosol® 250 HHR from Herkules. In an embodiment, the polysaccharide is selected from the group consisting of hydroxyethylcellulose, hydroxypropylcellulose, xanthan gum, carrageenans, and mixtures thereof. Xanthan gums and derivatives thereof may be present in an amount of from about 0.2% to about 1.5%, or from about 0.5% to about 0.9%, by total weight of the composition. Starches and derivatives thereof may be present in an amount of from about 3% to about 4% by total weight of the composition. In an embodiment, the viscosity-increasing agent is a starch compound. A suitable starch compound is hydroxypropyl starch phosphate such as Structure®

XL from National Starch. In an embodiment, the composition comprises two different polysaccharide viscosity-increasing agents.

The composition comprises a cosmetically acceptable carrier. The composition may comprise at least about 60%, or at least about 70%, or from about 75% to about 95%, or from about 80% to about 90%, of a cosmetically acceptable carrier, by total weight of the composition. The carrier may comprise water. In an embodiment, the carrier comprises water, fatty alcohols, volatile organic alcohols, and mixtures thereof. In an embodiment, the carrier is water.

In an embodiment, the composition comprises an oxidising agent. In an embodiment, the oxidising agent is hydrogen peroxide. Indeed, the oxidising agent may function as an initiator for the polymerisation and thus mixed into the composition immediately prior to application onto hair. In an embodiment, the composition is substantially free of oxidising agents and/or initiators. This may be the case when the hair has already been treated with an oxidising agent and/or initiator. In an embodiment, the composition is substantially free of oxidising agents selected from the group consisting of: peroxides; persulfates; and mixtures thereof.

In an embodiment, the composition is substantially free of: a lactone or an alpha-methylene lactone compound. In an embodiment, the composition is substantially free of a mercuric compound. In an embodiment, the composition comprises about 5% or less of or is substantially free of at least one of or all of the following: reducing agent, transition metal, alcohol, ammonia, lactone compound, mercuric compound, plasticizer, surfactant, neutralizing agent, propellant, silicone compound, fatty ester, fatty alcohol, hydrocarbon comprising 14 or more carbon atoms, lanolin, vitamin, protein, preservative, dye, tint, bleach, colorant, sunscreen, gelling agent, anti-dandruff active, hair growth active, non-polymeric thickener including clay, perfume. In an embodiment, the composition comprises about 5% or less of or is substantially free of at least one or all of the following: reducing agent, transition metal, alcohol, ammonia, lactone compound, mercuric compound, plasticizer, surfactant, neutralizing agent, propellant, silicone compound, fatty ester, fatty alcohol, hydrocarbon comprising 14 or more carbon atoms. Oily compounds may aid the penetration of the ethylenic monomer into the skin and/or scalp, which may not be preferred. In an embodiment, the composition is substantially free of any oily compounds.

The composition has a pH of from about 2.6 to about 4.5. The pH of the cosmetic composition is important because of potential sensitisation of the scalp and potential damage to the hair. Indeed, a pH lower than about 2.6 is not recommended for the present invention. Thus the lower limit of pH 2.6 has the benefit of reduced scalp sensitisation and reduced hair damage compared to a pH lower than this. In an embodiment, the pH is from about 2.7 to about 4.0, or from about 2.9 to about 3.9, or from about 3.0 to about 3.8, or from about 3.2 to about 3.7, or from about 3.4 to about 3.6. In an embodiment, the pH is from about 2.7 to about 3.8. The composition does not exhibit an alkaline pH. In an embodiment, the composition comprises a buffer system. In an embodiment, the buffer system comprises an acid and its conjugate base. In an embodiment, the buffer system is a phosphate-based buffer system. In an embodiment, the acid of the buffer system is phosphoric acid. In an embodiment, the buffer system is phosphoric acid ($H_3PO_4$) and the salt of a hydrogen phosphate ion. In an embodiment, the buffer system is citric acid and sodium hydroxide.

In an embodiment, the composition comprises an oxygen scavenger. Oxygen scavenger may increase the rate of conversion from monomer into polymer. In an embodiment, the oxygen scavenger is ascorbic acid.

A composition or formulation as described herein, or a plurality thereof, may further comprise one or more optional components known or otherwise effective for use in hair care or personal care products, provided that the optional components are physically and chemically compatible with the essential components described herein, or do not otherwise unduly impair product stability, aesthetics, or performance. Non-limiting examples of such optional components are disclosed in International Cosmetic Ingredient Dictionary, Ninth Edition, 2002, and CTFA Cosmetic Ingredient Handbook, Tenth Edition, 2004, both of which are incorporated by reference herein in their entirety. Some non-limiting examples of such optional components are disclosed below, and include plasticizer, surfactant (which may be anionic, cationic, amphoteric or nonionic), neutralizing agent, propellant, silicone compound, fatty ester, fatty alcohol, hydrocarbon comprising 14 or more carbon atoms, lanolin, vitamin, protein, preservative, dye, tint, bleach, colorant, sunscreen, gelling agent, anti-dandruff active, hair growth active, non-polymeric thickener including clay, perfume. In an embodiment, a composition or formulation as described herein comprises from about 0.001 % to about 10%, or 0.01 % to 5%, or 0.1% to 1% of an optional component.

In an embodiment, the composition comprises: a) from about 2.5% to about 3.5% ethylenic monomer, wherein the ethylenic monomer has a molecular weight of from about 70 g/mol to about 105 g/mole; and wherein the ethylenic monomer is selected from the group consisting of: methacrylic acid, acrylic acid, and mixtures thereof; b) a cosmetically acceptable carrier; and wherein the composition has a pH of from about 2.7 to about 3.8; and wherein composition comprises a buffer system; and wherein the composition comprises an inhibitor compound selected from the group consisting of: 2-*tert*-butyl-4-hydroxy-anisole, 3-*tert*-butyl-4-hydroxy-anisole, and mixtures thereof; and wherein the composition comprises an oxygen scavenger.

2<sup>nd</sup> Aspect: Method

The second aspect relates to a method for modifying the internal region of a hair shaft, wherein the method comprises: (A) mixing the composition according to the first aspect with an initiator formulation to form a mixture; and then (B) applying the mixture onto hair.

An alternative embodiment of the second aspect relates to a method for modifying the internal region of a hair shaft, wherein the method comprises: (A) applying an initiator formulation to the hair; and then (B) allowing the initiator formulation to remain on the hair for a period of time y, wherein time y is from about 1 min to about 120 min, or from about 2 min to about 45 min, or from about 3 min to about 20 min, or from about 4 min to about 10 min; and then (C) de-wetting the hair; and then (D) applying the composition according to the first aspect to the hair; and then (E) allowing the composition to remain on the hair for a period of time x, wherein the time x is from 1 min to 120 min, or from 2 min to 45 min, or from 3 min to 20 min, or from 4 min to 10 min; and then (F) rinsing and/or washing the hair; and then (G) applying a finishing formulation to the hair.

In an embodiment, the de-wetting the hair comprises the application of an absorbent material to the hair such that wetness is transferred from the hair to the absorbent material and wherein the wetness comprises the cosmetically acceptable carrier. The absorbent material may be selected from the group consisting of: towel, absorbent paper, and combinations thereof. In an embodiment, the de-wetting the hair comprises allowing moisture to evaporate from the hair wherein the moisture comprises the cosmetically acceptable carrier. In an embodiment, the de-wetting the hair comprises towel drying the hair such that the oxidising formulation no longer drips from the hair. In an embodiment, the de-wetting the hair comprises removing superficial initiator formulation from the hair. In an embodiment, the de-wetting the hair does not comprise rinsing the initiator formulation from the hair. The de-wetting the hair may last for time z, wherein time z is from about 1 min to about 120 min, from about 2 min to about 45 min, from about 3 min to about 20 min, or from about 4 min to about 10 min.

In an embodiment, the method does not comprise treating the hair with a reducing agent either prior to, concomitantly with or following the step of applying the mixture onto hair. The purpose of the reducing agent is to reduce the chemistry of the hair. The inventors have found that this step is not needed in order to achieve the benefits of the invention and thus the advantage of not first reducing the chemisty of the hair is faster overall treatment time and improved hair health versus methods where a reducing agent is employed since reducing agents typically damage the hair structure, even when the hair is oxidised again later. The reducing agent may be from the group consisting of: glycolates including thioglycolates; sulphites; bisulfites; and mixtures thereof.

In an embodiment, the initiator formulation comprises an oxidising agent. In an embodiment, the initiator formulation comprises an oxidising agent, and wherein the initiator formulation comprises from about 1% to about 100%, or from about 50% to about 100%, or from about 85% to about 99% oxidising agent. In an embodiment, the oxidising agent is hydrogen peroxide. In an embodiment, the initiator formulation is in solid form. In an embodiment, the solid form is selected from the group consisting of: powder, granules, and combinations thereof. In an embodiment, both the composition and the initiator formulation are substantially free of oily compounds. In an embodiment, the oxidising agent is a peroxide, and the peroxide is present in an amount of from about 0.5% to about 5%, or from about 1% to about 4%, or from about 1.3% to about 3%, or from about 1.5% to about 3%. In an embodiment, the initiator formulation comprises a salt. In an embodiment, the salt is an aluminium salt, preferably an aluminium sulfate salt. In an embodiment, the initiator formulation comprises from about 1% to about 5%, or from about 1.5% to about 3% of a salt. When the initiator formulation comprises peroxide, the oxidising formulation may comprise a buffer system to stabilise the pH. Suitable buffers may also act as chelating agents. Chelation of transition metals, for example copper or iron from pipes which might be present in trace amounts in tap water, is important because peroxides are sensitive to cleavage by transition metals. Typical buffer systems comprise a strong acid and its weak conjugate base or a weak base and its conjugate acid. An example of a suitable buffer system is phosphoric acid and disodium phosphate. Another example of a suitable buffer system is citric acid and sodium hydroxide.

3<sup>rd</sup> Aspect: Process

The third aspect relates to a process for preparing a mixture for applying onto hair, wherein the process comprises mixing a composition, according to the first aspect, with an initiator formulation being in solid form. The first aspect is already described above, as is the initiator formulation being in solid form, and these descriptions apply to and are suitable for the 3<sup>rd</sup> aspect as well.

4<sup>th</sup> Aspect: Kit

The fourth aspect relates to a kit comprising (i) the composition of the first aspect; (ii) an initiator formulation. The first aspect is already described above as is an initiator formulation and these descriptions apply to and are suitable

for the 4th aspect as well.

5th Aspect: Use

The fifth aspect relates to the use of the composition according to the first aspect applying onto hair. The first aspect is already described above and this applies to and are suitable for the 5th aspect as well.

EXAMPLES

The following examples further describe and demonstrate embodiments within the scope of the present invention. The examples are given solely for the purpose of illustration and are not to be construed as limitations of the present invention.

| Cosmetic Composition | Example 1 | Example 2 |
|---|---|---|
| Acrylic acid | 4.00 | 4.00 |
| Cellosize HEC QP 4400 [1] | 0.20 | 0.20 |
| EDTA | 0.12 | 0.12 |
| Keltrol CG-T [2] | 1.00 | 1.00 |
| Sodium hydroxide | 0.60 | 1.10 |
| Phenoxethol [3] | 1.00 | 1.00 |
| PHB-Methylester [4] | 0.20 | 0.20 |
| Genapol® C 100 [5] | 0.70 | 0.70 |
| Cremophor EL [6] | 0.70 | 0.70 |
| Fragrance | 0.30 | 0.30 |
| Distilled water | QSP 100% | QSP 100% |
| Total | 100 | 100 |
| pH | 3.56 | 4.28 |
| KEY: 1 = Hydroxyethylcellulose; 2 = Xanthan Gum (high molecular weight heteropolysaccharide gum produced by a pure-culture fermentation of a carbohydrate with *Xanthomonas campestris*); 3 = 2-(phenoxy)ethanol; 4 = methyl paraben; 5 = Coceth-10 (Coconut oil alcohol, ethoxylated); 6 = PEG-35 Castor Oil. | | |

| Initiator Formulation | Example 1 | Example 2 | Example 3 |
|---|---|---|---|
| **Phase 1** | | | |
| Purified water | 48.00 | 48.00 | 48.00 |
| Disodium phosphate | 0.08 | 0.08 | 0.08 |
| Salicylic acid | 0.10 | 0.10 | 0.10 |
| **Phase 2** | | | |
| Purified water | 49.96 | 47.35 | 49.56 |
| Hydrogen peroxide | 1.80 | 2.00 | 2.20 |
| Aluminium sulfate octadecahydrate | - | 2.40 | - |
| Phosphoric acid | 0.06 | 0.07 | 0.06 |
| Total | 100.00 | 100.00 | 100.00 |

Phase 1 and phase 2 are created separately and then mixed together. The phase 1 components are mixed together and heated to 80°C until the salicylic acid is dissolved. Phase 1 is then stirred and cooled to 40°C. Then phase 2 is created and mixed in with phase 1 with stirring for 5 min.

EXPERIMENTAL

<u>NMR Experiments</u>

The kinetics of the free radical polymerisation of ethylenic monomer in aqueous media can be followed using $^1$H-NMR. Determination of the conversion from may be performed by comparison of the signals of the residual (unpolymerised) ethylenic monomer to a signal of a reference substance added to the reaction mixture before start of the reaction and/or by comparison to signals stemming from both polymer and ethylenic monomer. Triethylene glycol and sodium benzenesulfonate can be used as inert reference substances. The conversion is deduced by comparison of the integrals. When an inert reference substance is added, the conversion can be calculated according to Equation A:

$$\text{conversion}[\%] = \left(1 - \frac{\dfrac{i_{mono}}{n_{H,mono} \cdot X_{mono}}}{\dfrac{i_{ref}}{n_{H,ref} \cdot X_{ref}}}\right) \cdot 100$$

<u>Equation A</u>

wherein $i_{mono}$: integral of the signal from ethylenic monomer; $n_{H,\,mono}$: number of H atoms in signal from ethylenic monomer; $X_{mono}$: amount of ethylenic monomer before start of the reaction in moles; $i_{ref}$: integral of the signal from reference; $n_{H,\,ref}$: number of H atoms in signal from reference; $X_{ref}$: amount of reference in moles.

For calculation of the conversion by comparison of residual ethylenic monomer signals and combined ethylenic monomer and polymer signals, Equation B can be utilised:

$$\text{conversion}[\%] = \left(1 - \frac{\dfrac{i_{mono}}{n_{H,mono}}}{\dfrac{i_{comb}}{n_{H,comb}}}\right) \cdot 100$$

<u>Equation B</u>

wherein $i_{mono}$: integral of the signal from the ethylenic monomer; $n_{H,\,mono}$: number of H atoms in signal from ethylenic monomer; $i_{comb}$: integral of the combined signal from ethylenic monomer and polymer; $n_{H,\,comb}$: number of H atoms in combined signal from ethylenic monomer and polymer.

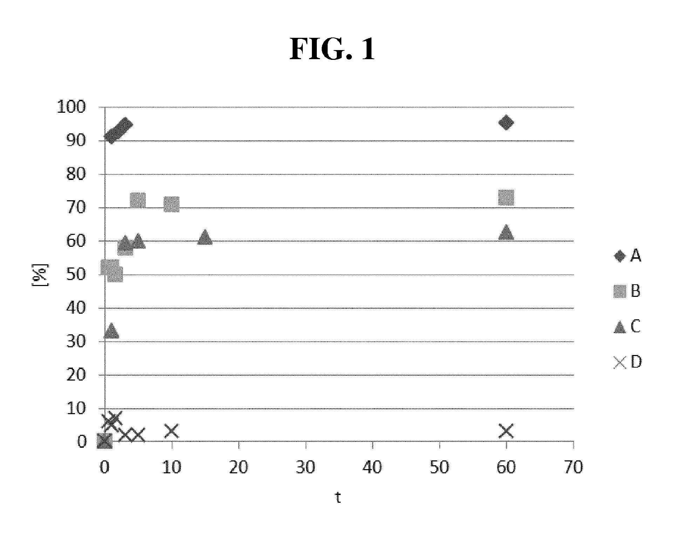

The following experimental procedure may be employed: ethylenic monomer and additives (when appropriate) in a round flask fitted with a silicone stopper are dissolved in 18 ml of the appropriate solvent (buffer with or without addition of salt, or ultra-pure demineralised water). The desired amount of initiator is dissolved in some ml of the same solvent. The reaction temperature is adjusted by an oil bath with contact thermometer. The reaction is started by transfer of the initiator solution to the reaction mixture with a syringe. In case no initiator is used, the flask with the starting materials is kept outside the oil bath before the reaction and the moment of contact with the oil bath is regarded as the starting point. At defined points in time, samples of 3.0 ml are drawn with a syringe. To stop the reaction, these samples are immediately exposed to air and 0.100 g of hydroquinone is added. The samples are freeze-dried separately and analysed by $^1$H-NMR spectroscopy for the determination of the conversion. The $^1$H-NMR spectroscopy measures the disappearance of a proton adjacent to the unsaturated carbon-carbon bond in the ethylenic monomer - this bond becomes saturated upon polymerisation and thus this proton no longer exhibits the same signal. A very similar experimental procedure and $^1$H-NMR spectroscopy method has already been described in WO2012/100006A and WO2012/100007A, for example. Utilising this technique, Fig. 1-3 herewith demonstrate the conversion of ethylenic monomer species into polymer species. The y axis labelled "[%]" shows conversion in percentage by mole of ethylenic monomer and the x-axis, which is labelled 't', shows time in min.

Fig. 1 relates to to the results of an experiment testing the effects of different pH of the composition on conversion of monomer to polymer and comparing 3-SPA with acrylic acid. The reactions are done in the presence of oxygen with the same weight percentage of ethylenic monomer in each case. In Fig. 1: A is 3-SPA (pH 2.3); B is acrylic acid (pH 2.3); C is arylic acid (pH 3.8); D is acrylic acid (pH 4.7). Conclusions drawn from Fig. 1 include that there is lower conversion of acrylic acid at all pH's relative to 3-SPA. A significantly improved conversion is seen when the pH of the samples comprising acrylic acid is reduced from pH 4.7 (sample D) to pH 3.8 (sample C).

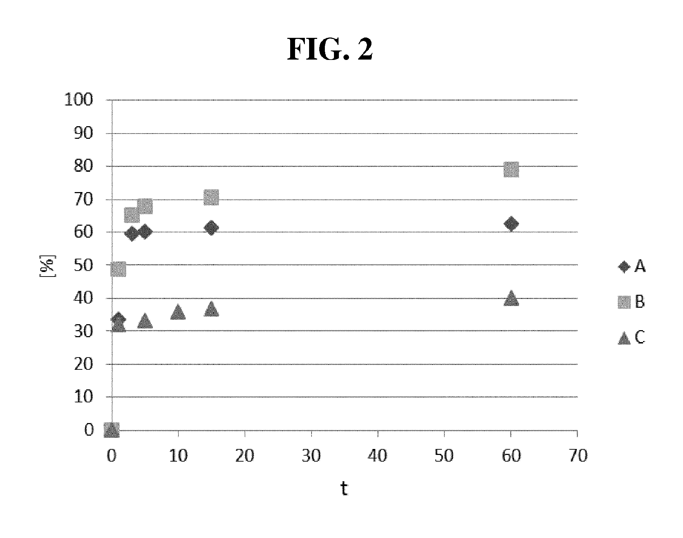

Fig. 2 relates to to the results of an experiment testing the effects of different pH of the composition on conversion of monomer to polymer. The monomer used is acrylic acid. The experiments are all done in the presence of $N_2$ gas rather than air (i.e. oxygen excluded) and all with the same amount of acrylic acid. In Fig. 2, A is at pH 2.3, B is at pH 3.8, C is at pH 4.5. Conclusions drawn from Fig. 2 include a improved conversion at about pH 3.8 versus at pH 2.3 and highly improved versus pH 4.5.

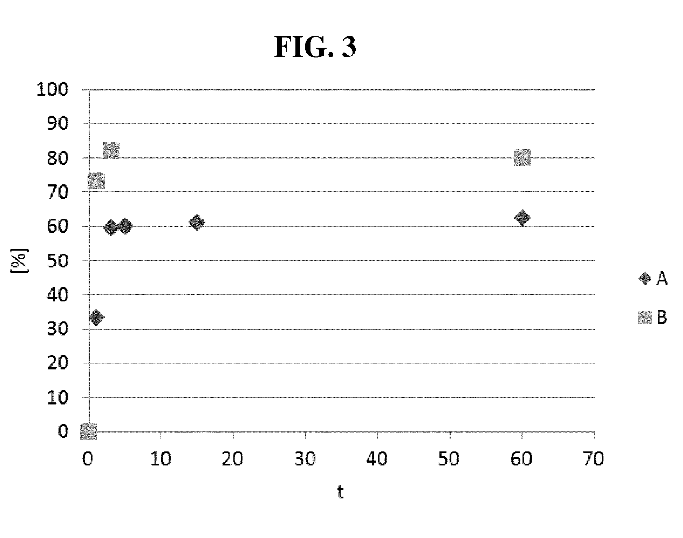

Fig. 3 relates to the results of an experiment testing the effects of a buffer system on conversion of monomer to polymer. The monomer used is acrylic acid. The experiments are all done in the presence of oxygen, both at pH 3.8 and the same amount of acrylic acid. In Fig. 3, sample B comprises a buffer system (1.5 mole/litre phosphate buffer) and A is the same sample without the buffer system (replaced with water). Conclusions drawn from Fig. 3 include that higher i.e. improved conversion of acrylic acid in the presence of a buffer system. Indeed, the presence of a buffer system improves the conversion by approximately 30%.

GPC Experiments

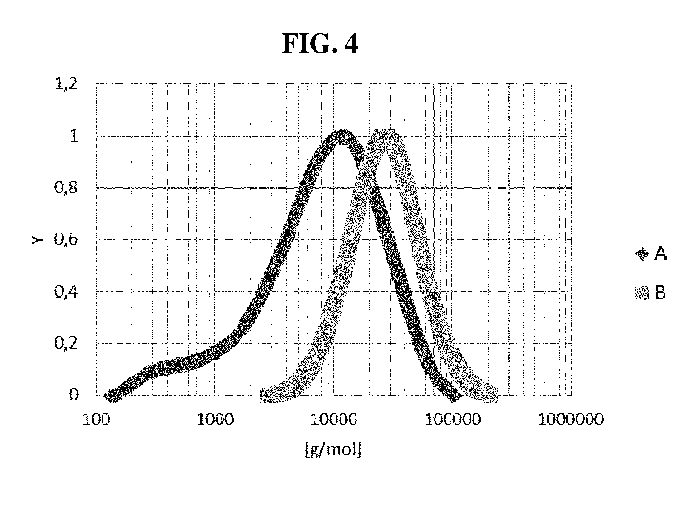

The M.Wt. of polymer resultant from the polymerisation of ethylenic monomers may be obtained by routine GPC in water-based (anionic) solution. The GPC equipment comprises a single-channel degassing system [WGE-Dr.Bures], an isocratic pump [P 1000 of Spectra Physics], GPC-liquid 0.1 mol/1 $NaNO_3$ at a flux rate of 1.0 ml/min, auto-sampler AS 1000 of Spectra Physics, sampling $100\mu$l, PSS SUPREMA column (1) Guard (8 x 50 mm), (2) Suprema 3000 A (8 x 300 mm; $10\mu$m), (3) Suprema 1000 A (8 x 300 mm; $10\mu$m), (4) Suprema 100 A (8 x 300 mm; $10\mu$m), column-oven K-7 of TECHLAB GmbH, Germany [at T=30°C], a UV - Detector UV 2000 of Spectra Physics, an RI-Detector Refractometer SEC-2010 of WGE Dr. Bures, Germany. Calibration was performed with Pullulan-Standards over a molecular weight range of 0.3 to 710 kDa. Data analysis was done using software WinGPC Unity by PSS. It is known in the art that the resulting polymer M.Wt. is inversely proportional to the square root of the initial radical concentration - a radical in this sense being a ethylenic monomer having been activated by an initiator. Thus, it is important to understand the kinetics of ethylenic monomer consumption in order to make reliable conclusions from the GPC data. Hence, the applicant has utilised $^1$H-NMR to follow the kinetics of ethylenic monomer conversion (see above). The GPC data is presented in Figures 4 to 6. The x-axis is the M.Wt. in g/mol (gram per mole). The y-axis represented by the letter 'Y' is the intensity.

In Fig. 4, the difference in M.Wt. of resulting polymer of a sample containing 3-SPA and a sample containing acrylic acid is tested. As per the graph, B = acrylic acid, pH is 3.8 (square marker); A = 3-SPA, pH 5.8 (diamond marker). Conclusions drawn from Fig. 4 thus include that a higher M.Wt. polymer results for a composition comprising acrylic acid versus for a composition comprising 3-SPA.

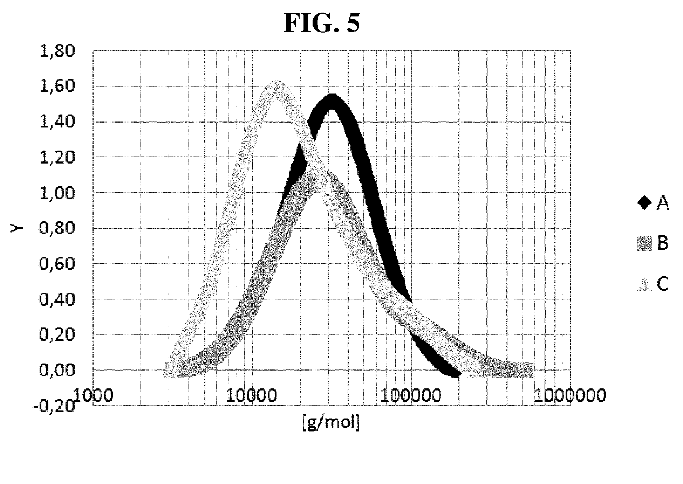

In Fig. 5, the difference in M.Wt. of resulting polymer of samples containing acrylic acid in compositions of differing pHs is tested. Only the pH differs between the samples and all experiments were done without the presence of oxygen (in $N_2$ gas). As per the graphs, A = acrylic acid at pH 2.3 (diamond, black marker); B = acrylic acid at pH 3.8 (square, grey marker); C = acrylic acid at pH 4.5 (triangle, lightest marker). Conclusions drawn from Fig. 5 thus include that lower M.Wt polymer results when the pH is at 4.5 versus the lower pHs of 2.3 and 3.8.

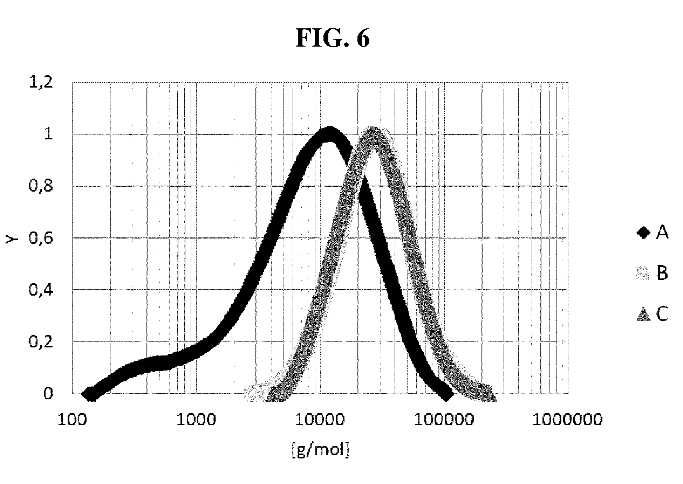

In Fig. 6, the difference in M.Wt. of resulting polymer of samples containing acrylic acid or 3-SPA in compositions with or without a buffer system is tested. All experiments were done in the presence of oxygen. As per the graphs, A = 3-SPA, at pH 5.8, without a buffer system (diamond, black marker); B = acrylic acid, pH 3.8, without a buffer system (square, lightest marker); C = acrylic acid, pH 3.8 with a buffer system (triangle, grey marker). Conclusions drawn from Fig. 6 thus include that higher M.Wt polymer results with acrylic acid and that the buffer system makes no difference to the M. Wt. of polymer resulting.

'Add on' Experiment

The change in weight of hair following treatment with a cosmetic composition according to the present invention, in this case comprising acrylic acid and at pH 3.56, was compared to the change in weight of hair following treatment with composition comprising 3-SPA and at pH 5.8. This 'add on' experiment correlates with the amount modification of the internal region of the hair shaft that occurs. Hair swatches were pre-prepared by bleaching twice and then allowing the swatches to rest at a relative humidity of 60%-70%, 22°C for 24 hours. The swatches were then treated with an initiator formulation comprising 2% hydrogen peroxide. Subsequently, 10 swatches were treated with the acrylic acid composition and 10 swatches with the 3-SPA composition. Other than this and as much as possible, the swatches were treated exactly the same. The swatches were first weighed to find the weight of each swatch before treatment. On damp hair swatches, a composition was applied in excess and worked in such that each swatch was fully coated and wetted. The swatches were then hung up to dry for 30 min at 45°C in a drying cabinet. Subsequently, the swatches were rinsed with 38°C running water and then washed with a neutral pH shampoo (0.25 ml of shampoo per gram of hair, 1 min shampooing time) and then well rinsed for 1 min. The swatches were then treated with a conditioning finishing formulation with a treatment time of 5 min, followed by rinsing with 38°C running water for 2 min. The swatches were then left to air dry. The swatches were then allowed to rest at a relative humidity of 60%-70%, 22°C for 24 hours. The swatches were then weighed to get the weight of each swatch after treatment. The mean change in weight for the 3-SPA treated swatches and for the acrylic acid treated swatches was calculated and converted to a percentage change.

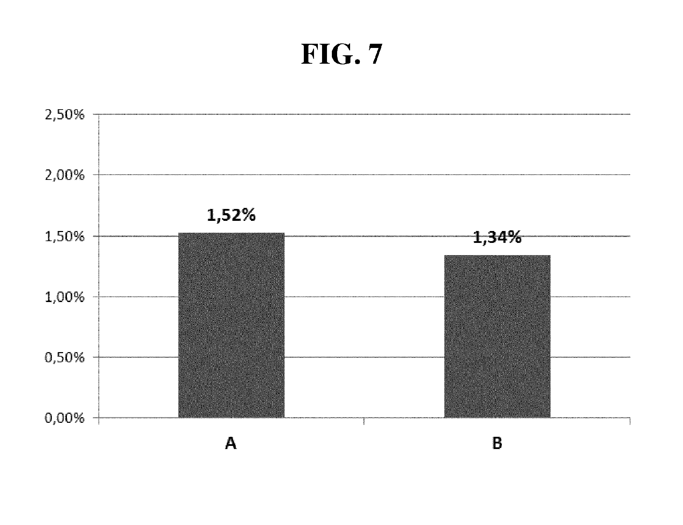

The results of the add on experiment are shown in Fig. 7. Conclusions from this experiment are that an increase in add on results for a composition comprising acrylic acid versus 3-SPA.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. A cosmetic composition for modifying the internal region of a hair shaft, wherein the composition comprises:

   a) from about 1.1% to about 4.9% ethylenic monomer, wherein the ethylenic monomer has a molecular weight of from about 50 g/mol to about 105 g/mole, and wherein the ethylenic monomer is an acid;
   b) a cosmetically acceptable carrier;
   wherein the composition has a pH of from about 2.6 to about 4.5.

2. The composition of claim 1, wherein the composition comprises a buffer system, wherein the buffer system comprises an acid and its conjugate base.

3. The composition of any of the preceding claims, wherein the pH is from about 2.7 to about 3.8.

4. The composition of any of the preceding claims, wherein the composition comprises about 5% or less of or is substantially free of at least one or all of the following: reducing agent, transition metal, alcohol, ammonia, lactone compound, mercuric compound, plasticizer, surfactant, neutralizing agent, propellant, silicone compound, fatty ester, fatty alcohol, hydrocarbon comprising 14 or more carbon atoms.

5. The composition of any of the preceding claims, wherein the ethylenic monomer has a molecular weight of from about 70 g/mol to about 100 g/mole.

6. The composition of any of the preceding claims, wherein the ethylenic monomer is selected from the group consisting of: methacrylic acid, tiglic acid, acrylic acid, and mixtures thereof.

7. The composition of any of the preceding claims, wherein the composition comprises an inhibitor compound selected from the group consisting of: 2-*tert*-butyl-4-hydroxy-anisole, 3-*tert*-butyl-4-hydroxy-anisole, and mixtures thereof.

8. The composition of any of the preceding claims, wherein the composition is substantially free of any nitrogen-containing ethylenic monomers.

9. The composition of any of the preceding claims, wherein the modifying is forming a polymer inside the hair shaft.

10. The composition of any of the preceding claims, wherein the composition comprises an oxygen scavenger.

11. A method for modifying the internal region of a hair shaft, wherein the method comprises: (A) mixing the composition according to any of claims 1 to 10 with an initiator formulation to form a mixture; and then (B) applying the mixture onto hair.

12. A method for modifying the internal region of a hair shaft, wherein the method comprises: (A) applying an initiator formulation to the hair; and then (B) allowing the initiator formulation to remain on the hair for a period of time y, wherein time y is from about 1 min to about 120 min, or from about 2 min to about 45 min, or from about 3 min to about 20 min, or from about 4 min to about 10 min; and then (C) de-wetting the hair; and then (D) applying the composition according to the first aspect to the hair; and then (E) allowing the composition to remain on the hair for a period of time x, wherein the time x is from 1 min to 120 min, or from 2 min to 45 min, or from 3 min to 20 min, or from 4 min to 10 min; and then (F) rinsing and/or washing the hair; and then (G) applying a finishing formulation to the hair.

13. A process for preparing a mixture for applying onto hair, wherein the process comprises mixing a composition, according to any of claims 1 to 10, with an initiator formulation being in solid form.

14. A kit comprising (i) the composition of any of claims 1 to 10; (ii) an initiator formulation.

15. The use of the composition according to any of claims 1 to 10 for applying onto hair.

# FIG. 1

# FIG. 2

# FIG. 3

# FIG. 4

FIG. 5

## FIG. 6

# FIG. 7

Europäisches Patentamt

European Patent Office

Office européen des brevets

## EUROPEAN SEARCH REPORT

**Application Number**

EP 13 15 7363

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 3 634 022 A (ROBBINS) 11 January 1972 (1972-01-11) | 1-6, 8-11, 13-15 | INV. A61K8/36 A61Q5/06 |
| Y | * column 4, line 3 - line 7 * <br> * examples 37, 39, 40 * <br> * page 12, line 34 - line 37 * <br> * column 7, line 15 - column 9, line 24 * <br> * column 10, line 64 - line 65 * <br> * examples 30,33, 38 * | 1-15 | |
| X | DATABASE GNPD [Online] MINTEL; August 2012 (2012-08), "Magneto Magnetic Gel Polish and Nail Lacquer", XP002716183, Database accession no. 1860707 * paragraph [ingredients] * | 1-6,8-10 | |
| X | FR 1 546 631 A (CLAIROL INC) 22 November 1968 (1968-11-22) | 1-6, 8-11,14, 15 | |
| | | | TECHNICAL FIELDS SEARCHED (IPC) |
| Y | * page 2, left-hand column, line 24 * <br> * page 2, left-hand column, line 25 - line 29 * <br> * page 2, right-hand column, line 7 - line 12 * <br> * examples 1, 3c, 4e * | 1-15 | A61K A61Q |
| X | EP 2 478 891 A1 (PROCTER & GAMBLE [US]) 25 July 2012 (2012-07-25) | 1-6,8-15 | |
| Y | * paragraphs [0007], [0081], [0046], [0051], [0052], [0007], [0045], [0048], [0056], [0083], [0049], [0050] * | 1-15 | |

-/--

The present search report has been drawn up for all claims

| Place of search | Date of completion of the search | Examiner |
|---|---|---|
| Munich | 13 November 2013 | Krattinger, B |

EPO FORM 1503 03.82 (P04C01)

| Europäisches Patentamt<br>European Patent Office<br>Office européen des brevets | **EUROPEAN SEARCH REPORT** | **Application Number**<br>EP 13 15 7363 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | G Blauer: "Polymerization of methacrylic acid at pH 4 to 11", Transactions of the Faraday Society, January 1960 (1960-01), pages 66-612, XP055087655, DOI: 10.1039/TF9605600606 Retrieved from the Internet: URL:http://pubs.rsc.org/en/content/article pdf/1960/tf/tf9605600606 [retrieved on 2013-11] * figure 1; table 2 * ----- | 1-6,8-10 | |
| X | A Katchalsky ET AL: "KINETICS OF METHACRYLIC ACID POLYMERIZA- TION IN AQUEOUS SOLUTION", , January 1951 (1951-01), pages 1360-1370, XP055087737, Retrieved from the Internet: URL:http://pubs.rsc.org/en/content/article pdf/1951/tf/tf9514701360 [retrieved on 2013-11-12] * paragraph [results/a] * ----- | 1-6,8-10 | |
| X | Z Zhao ET AL: "KINETICS O F POLYELECI'ROLYTE NETWORK FORMATION IN FREE-RADICAL COPOLYMERIZATION", Macromol. Symp, January 1995 (1995-01), pages 253-300, XP055087740, Retrieved from the Internet: URL:http://onlinelibrary.wiley.com/store/1 0.1002/masy.19950920122/asset/19950920122_ ftp.pdf?v=1&t=hnwv8qda&s=58338a478668563ef 0efa9183f9de5040b922e5b [retrieved on 2013-11-12] * abstract; figure 3 * ----- -/-- | 1-6,8-10 | TECHNICAL FIELDS SEARCHED (IPC) |

| The present search report has been drawn up for all claims | | |
|---|---|---|
| Place of search | Date of completion of the search | Examiner |
| Munich | 13 November 2013 | Krattinger, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EP 2 772 246 A1

| Europäisches Patentamt European Patent Office Office européen des brevets | **EUROPEAN SEARCH REPORT** | Application Number EP 13 15 7363 |
|---|---|---|

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ITO H, SHIMUZU S, SUZUKI S:<br>J. CHEM. SOC. JAPAN, IND. CHEM. SECT.,<br>vol. 58, no. 3, 1955, pages 194-196,<br>XP009174159,<br>* the whole document *<br>----- | 1-6,8-10 | |
| | | | **TECHNICAL FIELDS SEARCHED (IPC)** |

The present search report has been drawn up for all claims

| Place of search | Date of completion of the search | Examiner |
|---|---|---|
| Munich | 13 November 2013 | Krattinger, B |

EPO FORM 1503 03.82 (P04C01)

## ANNEX TO THE EUROPEAN SEARCH REPORT
## ON EUROPEAN PATENT APPLICATION NO.

EP 13 15 7363

This annex lists the patent family members relating to the patent documents cited in the above-mentioned European search report.
The members are as contained in the European Patent Office EDP file on
The European Patent Office is in no way liable for these particulars which are merely given for the purpose of information.

13-11-2013

| Patent document cited in search report | | Publication date | Patent family member(s) | | Publication date |
|---|---|---|---|---|---|
| US 3634022 | A | 11-01-1972 | BE | 751185 A1 | 03-11-1970 |
| | | | CA | 958336 A1 | 26-11-1974 |
| | | | CH | 547908 A | 11-04-1974 |
| | | | DE | 2025452 A1 | 28-01-1971 |
| | | | DK | 129023 B | 12-08-1974 |
| | | | ES | 379573 A1 | 16-10-1972 |
| | | | FR | 2043767 A1 | 19-02-1971 |
| | | | GB | 1321331 A | 27-06-1973 |
| | | | IT | 1013005 B | 30-03-1977 |
| | | | NL | 7007859 A | 01-12-1970 |
| | | | NO | 134184 B | 24-05-1976 |
| | | | PH | 11259 A | 02-11-1977 |
| | | | US | 3634022 A | 11-01-1972 |
| | | | ZA | 7002931 A | 29-12-1971 |
| FR 1546631 | A | 22-11-1968 | NONE | | |
| EP 2478891 | A1 | 25-07-2012 | NONE | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009088520 A
- WO 2012100006 A
- WO 2012100007 A
- EP 2295029 A
- US 3472243 A